(19) Europäisches Patentamt
European Patent Office
Office européen des brevets

(11) Veröffentlichungsnummer: **0 046 979**
**B1**

# (12) EUROPÄISCHE PATENTSCHRIFT

(45) Veröffentlichungstag der Patentschrift: **07.09.83**

(51) Int. Cl.³: **C 07 C 103/52**, A 61 K 37/26, C 12 P 21/02

(21) Anmeldenummer: **81106625.7**

(22) Anmeldetag: **26.08.81**

## (54) Analoga des Insulins.

(30) Priorität: **03.09.80 DE 3033127**

(43) Veröffentlichungstag der Anmeldung: **10.03.82 Patentblatt 82/10**

(45) Bekanntmachung des Hinweises auf die Patenterteilung: **07.09.83 Patentblatt 83/36**

(84) Benannte Vertragsstaaten: **AT BE CH DE FR GB IT LI NL SE**

(56) Entgegenhaltungen:
**DE-B-2 005 658**
**US-A-3 364 116**
**NATURE, Band 280, Nr. 5721, 1979 Basingstoke MORIHARA et al. »Semisynthesis of human insulin by trypsin-catalysed replacement of Ala-B30 by Thr in porcine insulin« Seiten 412 bis 413**

(73) Patentinhaber: **HOECHST AKTIENGESELLSCHAFT, Postfach 80 03 20, D-6230 Frankfurt am Main 80 (DE)**

(72) Erfinder: **Geiger, Rolf, Prof. Dr., Heinrich-Bleicher-Strasse 33, D-6000 Frankfurt am Main 50 (DE)**

**EP 0 046 979 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Analoga des Insulins

Die Erfindung betrifft Insuline der Formel I

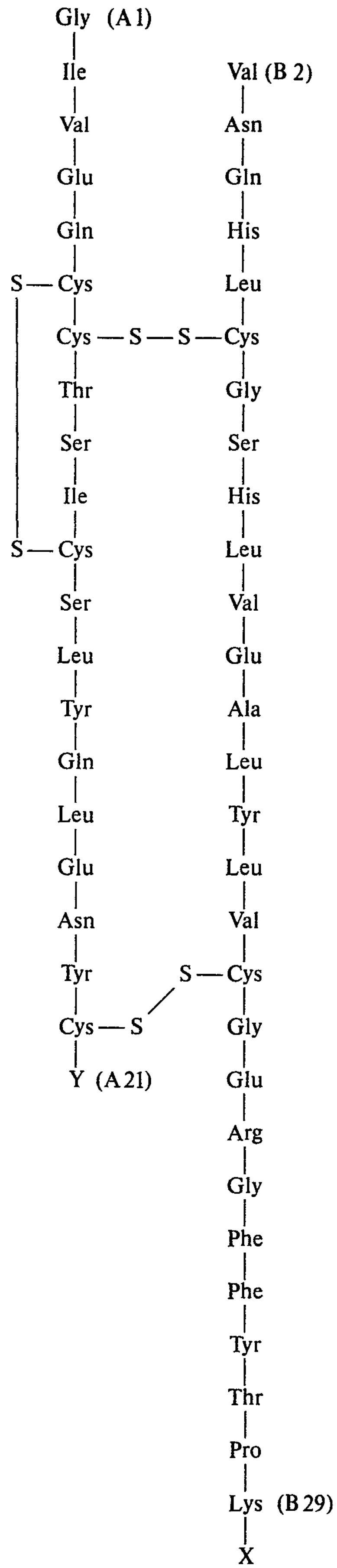

Die neuen Verbindungen besitzen mit Ausnahme einer geringfügigen Veränderung die Aminosäuresequenz des Humaninsulins.

Insuline mit fehlendem B1-Phenylalanin sind bereits bekannt, jedoch ist das $Des^{B1}$-Phenylalanin-humaninsulin (des $Phe^{B1}$-humaninsulin) bisher noch nicht beschrieben worden.

Nachdem nunmehr Humaninsulin als Ausgangsprodukt für Versuchszwecke auf mehreren Wegen hergestellt werden kann, war es erstmals möglich, die vorteilhaften Eigenschaften von Des-$Phe^{B1}$-humaninsulin (Formel I mit X = Thr, Y = Asn) zu erkennen.

Bei den erfindungsgemäßen Verbindungen mit X = Thr ist ganz allgemein die Löslichkeit gegenüber den Verbindungen, die noch $Phe^{B1}$ enthalten, beträchtlich erhöht, so daß hochkonzentrierte Lösungen hergestellt werden können wie sie für Insulinpumpen benötigt werden.

Darüber hinaus besitzen diese Verbindungen Vorzüge auf immunologischem Gebiet insofern, als sie in geringem Maße die Bildung von Antikörpern hervorrufen. Bei der Langzeitbehandlung mit Insulinen spielt die Erzeugung von Antikörpern eine kritische Rolle, die sogar durch homologes Insulin induziert werden kann. So ist z. B. beobachtet worden, daß auch Humaninsulin beim Menschen Antikörper zu erzeugen vermag.

Ein empfindliches Testmodell für die Erzeugung von Antikörpern ist die Ziege, die in Anwesenheit von Freund'schem Adjuvans gegen Schweineinsulin und gegen Humaninsulin sehr rasch Antikörper bildet. Nach Diabetes 27 (1978), Seite 14, Abb. 4 ist die Bindekapazität von Antikörpern gegen das heterologe Des-$Phe^{B1}$-insulin vom Schwein niedriger und die Antikörperbildung erfolgt langsamer als beim unveränderten Schweineinsulin oder selbst beim homologen Schafinsulin. Die erfindungsgemäßen Verbindungen, vor allem die mit X = OH, bewirken in diesem Testmodell eine weiter verlangsamte Antikörperbildung und die Bindekapazität gegenüber der von Des-$Phe^{B1}$-insulin vom Schwein wird nochmals um 15% verringert.

Ähnlich wie Humaninsulin zeigen auch die erfindungsgemäßen Verbindungen bei der Behandlung des Menschen gegenüber dem Schweineinsulin den Vorteil einer besseren Glucosetoleranz. Diese Eigenschaft wird offensichtlich durch die Abspaltung von $Phe^{B1}$ nicht verändert.

Darüber hinaus verfügt insbesondere das Des-$Phe^{B1}$-Humaninsulin (I mit X = Thr) über eine rasch einsetzende und lange anhaltende Wirkung.

Spaltet man zusätzlich zu $Phe^{B1}$ aus dem Humaninsulin noch $Thr^{B30}$ (I mit X = OH) enzymatisch ab, so bleiben alle genannten vorteilhaften Eigenschaften des Des-$Phe^{B1}$-humaninsulins erhalten. Dasselbe gilt für die Verbindung, die anstelle von $Asn^{A21}$ mit $Asp^{A21}$ eine zusätzliche Carboxylgruppe trägt (I mit X = OH, Y = Asp). In diesem Fall tritt sogar eine weitere Erhöhung der Löslichkeit ein, die vor allem im neutralen und schwach alkalischen Bereich sichtbar wird.

Der besondere ökonomische Vorteil der Verbindungen I mit X = OH liegt darin, daß bei der Herstellung nicht das zur Zeit noch immer sehr schwer zugängliche Humaninsulin eingesetzt werden muß, sondern Schweineinsulin verwendet werden kann. Schweineinsulin unterscheidet sich von Humaninsulin nur in der Aminosäure $B^{30}$ (Ala statt Thr) und wird nach Abspalten dieser Aminosäure mit Des-$Thr^{B30}$-humaninsulin identisch.

Die Abspaltung von $Ala^{B30}$ aus Schweineinsulin ist schon in der US-Patentschrift 3 364 116 beschrieben worden. Des-$Ala^{B30}$-Schweineinsulin unterscheidet sich aber hinsichtlich seiner immunologischen Eigenschaften nur wenig von Schweineinsulin. Dagegen löst die zusätzliche Entfernung von $Phe^{B1}$ strukturelle Veränderungen im Insulinmolekül aus, die für die vorteilhaften biologischen Eigenschaften verantwortlich sind.

Gegenstand der Erfindung ist auch ein Verfahren zur Herstellung der Insuline der Formel I. Dieses ist dadurch gekennzeichnet, daß man

a) Humaninsulin
   $Desamido^{A21}$-Humaninsulin
   Des-$Thr^{B30}$-Humaninsulin
   $Desamido^{A21}$-des-$Thr^{B30}$-Humaninsulin
   Des-$Ala^{B30}$-Schweineinsulin oder
   $Desamido^{A21}$-des-$Ala^{B30}$-Schweineinsulin
   in die $N^{\alpha A1}$-$N^{\varepsilon B29}$-Bis-boc-Verbindung überführt und diese einem Edman-Abbau unterwirft,
b) Des-$Phe^{B1}$-Humaninsulin oder
   Des-$Phe^{B1}$-des-$Ala^{B20}$-Schweineinsulin
   mit Säure behandelt, oder
c) eine Verbindung der Formel I, in der X Ala bedeutet, mit Carboxypeptidase A behandelt.

Zur Herstellung der erfindungsgemäßen Verbindungen mit X = Thr und Y = Asn geht man von Humaninsulin aus, das analog dem DBP 2 005 658 in die entsprechende Des-$Phe^{B1}$-Verbindung übergeführt wird.

Zur Herstellung der Verbindung I mit X = OH und Y = Asn geht man von Des-$Ala^{B30}$-Schweineinsulin aus, hergestellt z. B. nach Hoppe-Seyler's Z. Physiol. Chem. 359 (1978), Seite 799, durch enzymatische Abspaltung von $Ala^{B30}$ und spaltet $Phe^{B1}$ analog dem DBP 2 005 658 ab. Man kann die Reihenfolge der Schritte auch umkehren und zunächst $Phe^{B1}$ chemisch und anschließend $Ala^{B30}$ enzymatisch abspalten.

Soll Y=Asp sein, so werden die Ausgangsverbindungen zunächst in bekannter Weise bei pH 2–3 mit wäßriger Säure behandelt und durch Ionenaustauschchromatographie gereinigt. $Phe^{B1}$ bzw. $Ala^{B30}$ werden sodann wie oben beschrieben entfernt.

Man kann die Säurebehandlung auch nach Abspalten von $Phe^{B1}$ und gegebenenfalls $Ala^{B30}$ vornehmen. Dies hat den Vorteil, daß bei der Endreinigung durch Ionenaustauscherchromatographie ein sehr einheitliches Produkt erscheint. Man kommt so mit 1–2 Reinigungsoperationen aus, während man beim umgekehrten Verfahren meist einen weiteren Reinigungsschritt benötigt.

Die neuen Humaninsulin-Analoga werden zur Behandlung des Diabetes mellitus eingesetzt und können wegen ihrer sehr guten Löslichkeit bei pH 7–7,5 auch in der Insulinpumpe in einer Konzentration von etwa 10% verwendet werden. Die Dosierung hängt von der Einstellung des Patienten ab und entspricht etwa der von Schweineinsulin, kann aber bei Patienten mit erhöhtem Antikörpertiter wesentlich niedriger liegen.

Ein weiterer Gegenstand der Erfindung ist eine pharmazeutische Zubereitung der Verbindungen der Formel I, enthaltend eine Verbindung der Formel I in gelöster oder amorpher Form neben einer Verbindung der Formel I, die jedoch zusätzlich Phenylalanin in B1 enthält, in kristalliner Form.

Die neuen Humaninsulin-Analoga können natürlich auch zusammen mit Verbindungen verwendet werden, die $Phe^{B1}$ noch enthalten. Sie liegen dann in gelöster oder amorpher Form vor während die $Phe^{B1}$-haltigen Verbindungen kristallin eingesetzt werden. Zweiphasige Insuline, bei denen rascher Wirkungseintritt mit langer Wirkdauer verbunden ist, sind an sich bekannt. Sie sind z. B. in der DOS 2 459 515 (HOE 74/F 377) und der DOS 2 256 215 (HOE 72/F 348) beschrieben. Die neuen erfindungsgemäßen Zubereitungen werden analog den dort für Schweineinsulin gegebenen Vorschriften hergestellt. Eine andere Zubereitungsform, bei der eine Verbindung der Formel I mit Y=Asp in Lösung neben der entsprechenden mit Y=Asn in kristalliner Form vorliegt, ist ebenfalls Gegenstand der Erfindung. Sie kann analog der DOS 2 803 996 hergestellt werden.

## Beispiele

Die Charakterisierung der Zwischen- und Endprodukte erfolgte durch Papier- und Gel-Elektrophorese bei Ph 2 und 8,3, durch Aminosäureanalyse, Dünnschichtchromatographie und HPLC in den bekannten Systemen.

### Beispiel 1

### Des-$Phe^{B1}$-humaninsulin

a) 1 g Humaninsulin werden in 70 ml 80prozentigem, wäßrigem Dimethylacetamid aufgenommen. Man gibt 1,45 g tert.-Butyloxycarbonylazid und 3,3 ml 1 N Natriumhydrogencarbonat zu und rührt 5 Stunden bei 35°C. Dann wird die Lösung bei höchstens 50°C Badtemperatur eingeengt. Den Rückstand verreibt man mit Ether und digeriert ihn mit 10 ml 2prozentiger Essigsäure. Ausbeute 942 mg $N^{\alpha A1}$, $N^{\varepsilon B29}$-Di-tert.-Butyloxycarbonylinsulin vom Menschen.

b) Die erhaltene Verbindung wird in 4 ccm 95prozentigem Pyridin gelöst. Man gibt 0,03 ccm Phenylisothiocyanat zu und rührt 4 Stunden bei Raumtemperatur. Dann wird im Vakuum bei höchstens 50°C Badtemperatur auf ein kleines Volumen eingeengt und die Verbindung mit Äther ausgefällt.
Ausbeute: 830 mg $N^{\alpha(A1)}$, $N^{\varepsilon(B29)}$-Di-tert.-Butyloxycarbonyl-$N^{\alpha(B1)}$-phenylthiocarbamoyl-humaninsulin

c) 820 mg der nach b) hergestellten Verbindung werden 1 Stunde in 8,5 ccm Trifluoressigsäure bei Raumtemperatur aufbewahrt. Auf Zusatz von 100 ccm Äther fallen 725 mg Des-$Phenylalanin^{B1}$-humaninsulin aus, die in bekannter Weise bei pH 5,0 bis 5,5 zur Kristallisation gebracht werden können.
Phe Ber. 2,00, gef. 2,02

### Beispiel 2

### Des-$Phe^{B1}$-des-$Thr^{B30}$-humaninsulin

a) 1,0 g Des-$Ala^{B30}$-schweineinsulin, hergestellt nach Hoppe-Seyler's Z. Physiol. Chem. 359 (1978), Seite 799, werden analog Beispiel 1a–c umgesetzt. Man erhält 710 mg der Titelverbindung, die durch Chromatographie an einer Säule Sephadex® G 50 superfein 1×100 cm in 1prozentiger Essigsäure gereinigt werden kann.
Ausbeute 623 mg. Kristallisation bei pH 5,4.
Phe Ber. 2,00, gef. 1,99

b) 500 mg Des-$Phe^{B1}$-schweineinsulin, hergestellt analog Beispiel 1 aus Schweineinsulin, werden in 100 ml 0,1 M Ammoniumhydrogencarbonatpuffer bei pH 8,2 gelöst. Man gibt 3 mg Carboxypeptidase A zu, hält 15 Stunden bei Raumtemperatur und lyophilisiert dann. Zur Entfernung von Salzen wird in 1prozentiger Essigsäure über eine Sephadex® G 15-Säule 1 × 50 cm chromatographiert. Zur weiteren Reinigung wurde in 30prozentigem Isopropanol mit 0,05 M Tris-Puffer bei pH 8 und einem NaCl-Gradienten von 0 bis 0,25 M in einer Säule 2,5 × 50 cm DEAE-Sephadex®A-25 chromatographiert. Das Eluat wurde durch Dialyse gegen destilliertes Wasser entsalzt und lyophilisiert.
Ausbeute 276 mg.
Phe Ber. 2, gef. 1,98; Ala Ber. 1, gef. 1,01

Beispiel 3

Des-$Phe^{B1}$-[$Asp^{A21}$]humaninsulin

a) 0,5 g [$Asp^{A21}$]Humaninsulin, hergestellt durch 3tägiges Aufbewahren von Humaninsulin in wäßriger Trifluoressigsäure bei pH 2, Gefriertrocknen und Reinigung durch Chromatographie an DEAE-Sephadex®A-25 analog Beispiel 2b) werden analog Beispiel 1a – c) umgesetzt. Man erhält nach Chromatographie analog Beispiel 2a) 324 mg der Titelverbindung.
Phe Ber. 2, gef. 2,02
Charakterisierung in der Gel-Elektrophorese bei pH 8. Die Laufstrecke entspricht der von Desamidoinsulin (geringfügige Abweichung durch das fehlende Phenylalanin).

b) 0,5 g Des-$Phe^{B1}$-humaninsulin, hergestellt nach Beispiel 1, werden wie unter a) beschrieben in wäßriger Trifluoressigsäure aufbewahrt und anschließend an DEAE-Sephadex®A-25 gereinigt. Man kristallisiert in bekannter Weise bei pH 5,0 und erhält 365 mg der Titelverbindung, die mit der nach a) hergestellten identisch ist.

Beispiel 4

Des-$Phe^{B1}$-des-$Thr^{B30}$-[$Asp^{A21}$]humaninsulin

0,5 g Des-$Phe^{B1}$-des-$Thr^{B30}$-humaninsulin, hergestellt nach Beispiel 2, werden nach Beispiel 3b) mit Säure behandelt und gereinigt.
Ausbeute 344 mg
Phe Ber. 2, gef. 1,99; Ala Ber. 1, gef. 1,02
Weitere Charakterisierung durch Gelelektrophorese bei pH 8 analog Beispiel 3a).

**Patentansprüche für die Vertragsstaaten: BE, CH, LI, DE, FR, GB, IT, NL, SE**

1. Insulin der Formel I

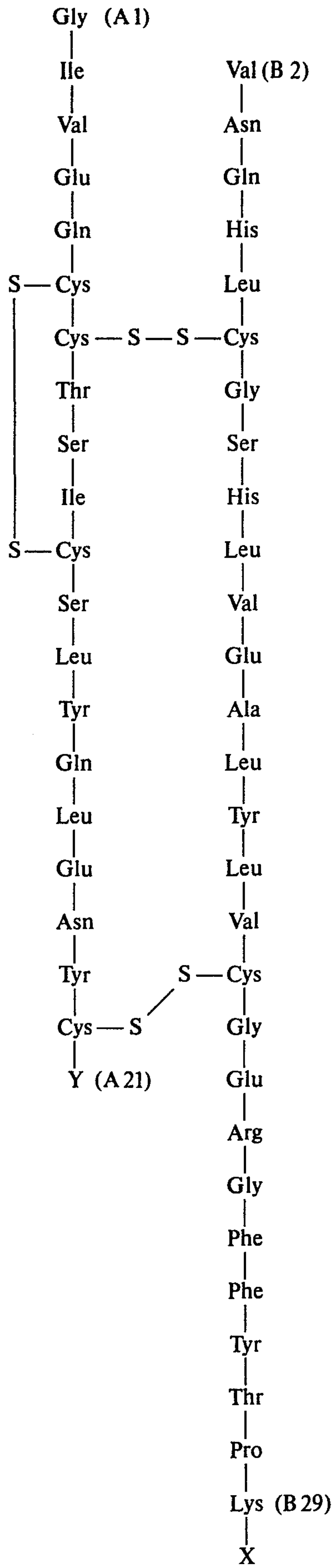

worin X Thr oder OH und Y Asp oder Asn bedeuten.

2. Verfahren zur Herstellung eines Insulins der Formel I, dadurch gekennzeichnet, daß man

a) Humaninsulin
Desamido$^{A21}$-Humaninsulin
Des-Thr$^{B30}$-Humaninsulin
Desamido$^{A21}$-des-Thr$^{B30}$-Humaninsulin
Des-Ala$^{B30}$-Schweineinsulin oder
Desamido$^{A21}$-des-Ala$^{B30}$-Schweineinsulin
in die $N^{\alpha A1}$-$N^{\varepsilon B29}$-Bis-Boc-Verbindung überführt und diese einem Edman-Abbau unterwirft,

b) Des-Phe$^{B1}$-Humaninsulin oder
Des-Phe$^{B1}$-des-Ala$^{B20}$-Schweineinsulin
mit Säure behandelt, oder

c) eine Verbindung der Formel I, in der X Ala bedeutet, mit Carboxypeptidase A behandelt.

3. Pharmazeutische Zubereitung enthaltend eine Verbindung der Formel I in gelöster oder amorpher Form neben einer Verbindung der Formel I, die jedoch zusätzlich Phenylalanin in B1 enthält, in kristalliner Form.

4. Pharmazeutische Zubereitung enthaltend eine Verbindung der Formel I mit Y = Asparaginsäure in gelöster Form und eine Verbindung der Formel I mit Y = Asparagin in kristalliner Form.

**Patentansprüche für den Vertragsstaat: AT**

1. Verfahren zur Herstellung eines Insulins der Formel I

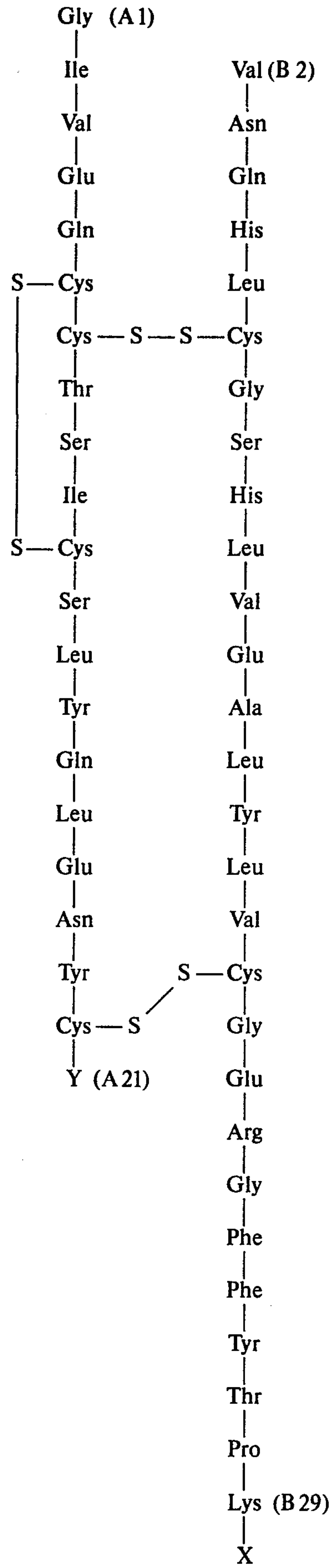

worin X Thr oder OH und Y Asp oder Asn bedeuten, dadurch gekennzeichnet, daß man

a) Humaninsulin
Desamido$^{A21}$-Humaninsulin
Des-Thr$^{B30}$-Humaninsulin
Desamido$^{A21}$-des-Thr$^{B30}$-Humaninsulin
Des-Ala$^{B30}$-Schweineinsulin oder
Desamido$^{A21}$-des-Ala$^{B30}$-Schweineinsulin
in die $N^{\alpha A1}$-$N^{\varepsilon B29}$-Bis-Boc-Verbindung überführt und diese einem Edman-Abbau unterwirft,

b) Des-Phe$^{B1}$-Humaninsulin oder
Des-Phe$^{B1}$-des-Ala$^{B20}$-Schweineinsulin
mit Säure behandelt, oder

c) eine Verbindung der Formel I, in der X Ala bedeutet, mit Carboxypeptidase A behandelt.

2. Verfahren zur Herstellung einer pharmazeutischen Zubereitung, dadurch gekennzeichnet, daß man eine Verbindung der Formel I in gelöster oder amorpher Form mit einer Verbindung der Formel I, die jedoch zusätzlich Phenylalanin in B1 enthält, in kristalliner Form zusammengibt.

3. Verfahren zur Herstellung einer pharmazeutischen Zubereitung, dadurch gekennzeichnet, daß man eine Verbindung der Formel I mit Y=Asparaginsäure in gelöster Form mit einer Verbindung der Formel I mit Y=Asparagin in kristalliner Form zusammengibt.

**Claims for contracting states: BE, LI, DE, FR, GB, IT, NL, SE**

1. Insulin of the formula I

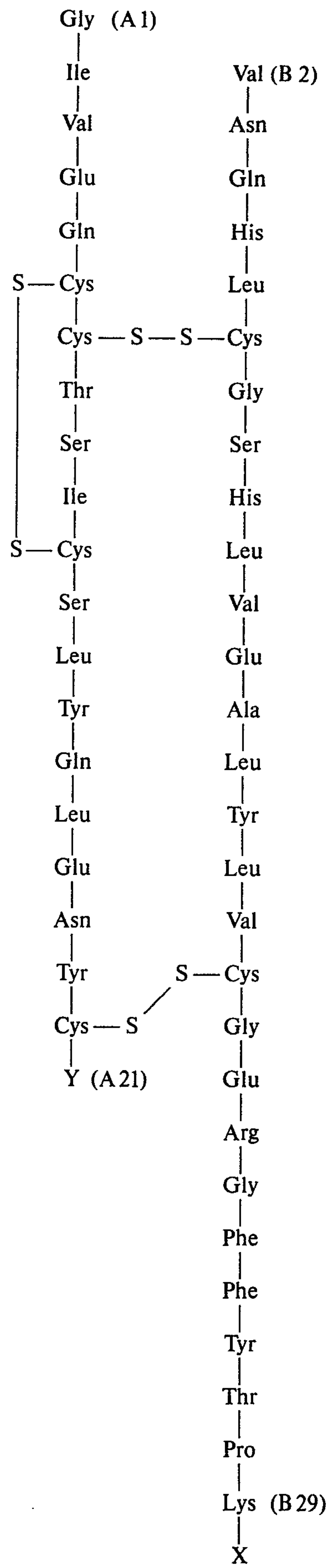

in which X denotes Thr or OH and Y denotes Asp or Asn.

2. A process for the preparation of an insulin or the formula I, which comprises a) converting human insulin, desamido$^{A21}$-human insulin, des-Thr$^{B30}$-human insulin, desamido$^{A21}$-des-Thr$^{B30}$-human insulin, des-Ala$^{B30}$-swine insulin or desamido$^{A21}$-des-Ala$^{B30}$-swine insulin into the $N^{\alpha A1}$-$N^{\epsilon B29}$-bis-Boc compound and subjecting this to Edman degradation, b) treating des-Phe$^{B1}$-human insulin or des-Phe$^{B1}$-des-Ala$^{B20}$-swine insulin with acid, or c) treating a compound of the formula I in which X denotes Ala with carboxypeptidase A.

3. A pharmaceutical formulation containing a compound of the formula I in dissolved or amorphous form, in addition to a compound of the formula I, in crystalline form, which however, additionally contains phenylalanine in BI.

4. A pharmaceutical formulation containing a compound of the formula I in which Y=aspartic acid, in dissolved form and a compound of the formula I, in which Y=asparagine, in crystalline form.

**Claims for contracting states: AT**

1. A process for the preparation of an insulin of the formula I,

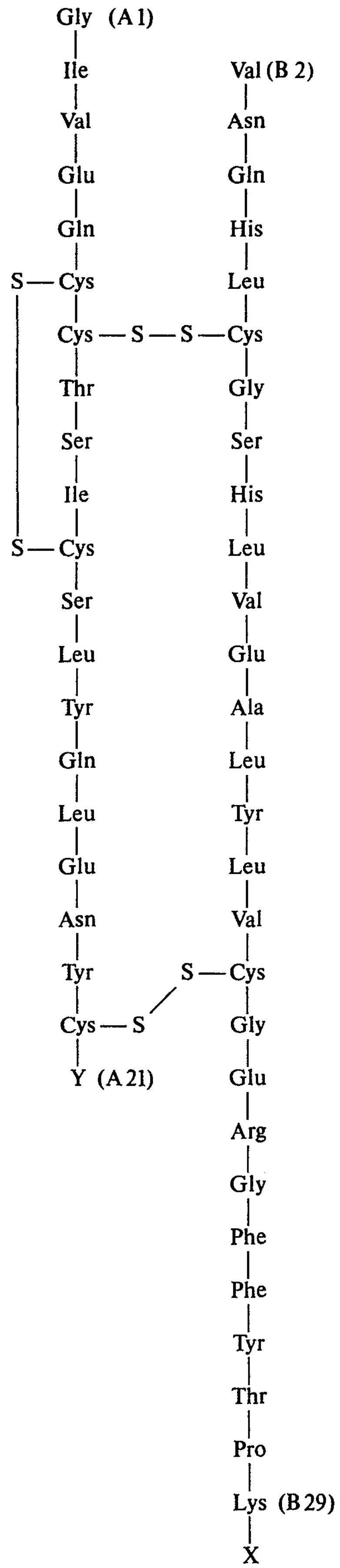

in which X denotes Thr or OH and Y denotes Asp or Asn, which comprises a) converting human insulin,

desamido$^{A21}$-human insulin, des-Thr$^{B30}$-human insulin, desamido$^{A21}$-des-Thr$^{B30}$-human insulin, des-Ala$^{B30}$-swine insulin or desamido$^{A21}$-des-Ala$^{B30}$-swine insulin into the $N^{\alpha AI}$-$N^{\varepsilon B29}$-bis-Boc compound and subjecting this to Edman degradation, b) treating des-Phe$^{BI}$-human insulin or des-Phe$^{BI}$-des-Ala$^{B20}$-swine insulin with acid, or c) treating a compound of the formula I in which X denotes Ala with carboxypeptidase A.

2. A process for the preparation of a pharmaceutical formulation, characterized in that a compound of the formula I in dissolved or amorphous form, and a compound of the formula I, in crystalline form, which however, additionally contains phenylalanine in BI, are brought together.

3. A process for the preparation of a pharmaceutical formulation, characterized in that a compound of the formula I in which Y = aspartic acid, in dissolved form and a compound of the formula I, in which Y = asparagine, in crystalline form are brought together.

**Revendications pour les Etats contractants: BE, CH, LI, DE, FR, GB, IT, NL, SE**

1. Insuline répondant à la formule I

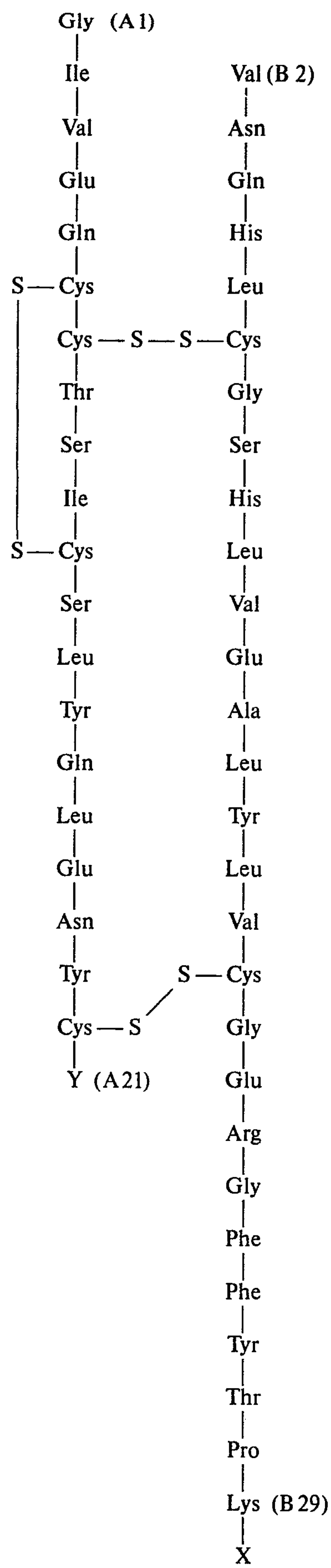

dans laquelle X représente Thr ou OH et Y représente Asp ou Asn.

2. Procédé pour la préparation d'une insuline de formule I, caractérisé en ce que:

a) on convertit
l'insuline humaine
la desamido$^{A21}$-insuline humaine
la des-Thr$^{B30}$-insuline humaine
la desamido$^{A21}$-des-Thr$^{B30}$-insuline humaine
la des-Ala$^{B30}$-insuline de porc ou
la desamido$^{A21}$-des-Ala$^{B30}$-insuline de porc
en le $N^{\alpha A1}$-$N^{\varepsilon B29}$-Bis-Boc-composé et on soumet ce dernier à une dégradation d'Edman,

b) on traite
la des-Phe$^{B1}$-insuline humaine ou
la des-Phe$^{B1}$-des-Ala$^{B20}$-insuline de porc
par un acide, ou

c) on traite un composé de formule I dans lequel X représente Ala, avec la carboxypeptidase A.

3. Composition pharmaceutique contenant un composé de formule I sous une forme dissoute ou amorphe, à côté d'un composé de formule I qui contient en outre la phénylalanine en BI, sous une forme cristalline.

4. Composition pharmaceutique contenant un composé de formule I avec Y = acide aspartique sous une forme dissoute et un composé de formule I où Y = asparagine sous une forme cristalline.

**Revendications pour l'Etat Contractant AT**

1. Procédé pour la préparation d'une insuline répondant à la formule I

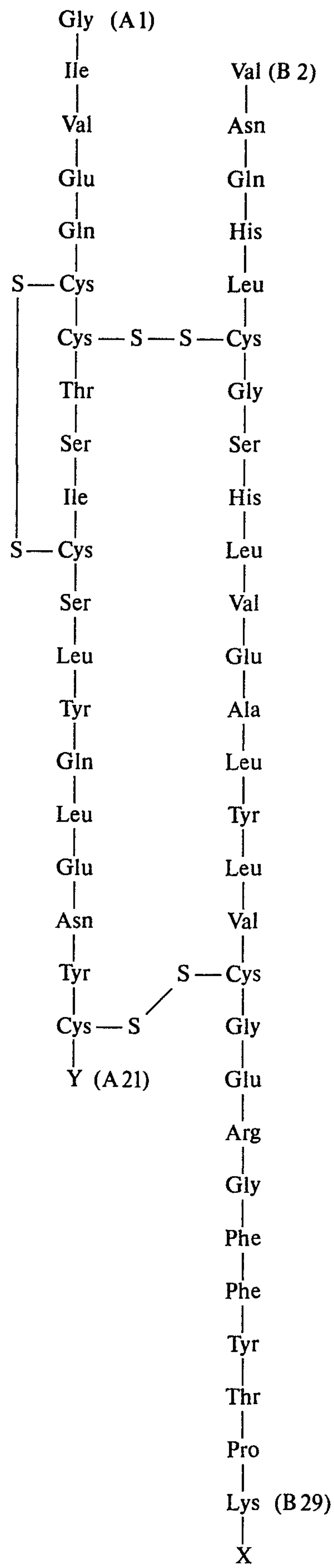

dans laquelle X représente Thr ou OH et Y représente Asp ou Asn, caractérisé en ce que:

a) on convertit
l'insuline humaine
la desamido$^{A21}$-insuline humaine
la des-Thr$^{B30}$-insuline humaine
la desamido$^{A21}$-des-Thr$^{B30}$-insuline humaine
la des-Ala$^{B30}$-insuline de porc ou
la desamido$^{A21}$-des-Ala$^{B30}$-insuline de porc
en le N$^{\alpha AI}$-N$^{\varepsilon B29}$-Bis-Boc-composé et on soumet ce dernier à une dégradation d'Edman,

b) on traite
la des-Phe$^{BI}$-insuline humaine ou
la des-Phe$^{BI}$-des-Ala$^{B20}$-insuline de porc
avec un acide, ou

c) on traite un composé de formule I, dans lequel X représente Ala, avec la carboxypeptidase A.

2. Procédé pour la préparation d'une composition pharmaceutique, caractérisé en ce qu'on réunit un composé de formule I sous une forme dissoute ou amorphe avec un composé de formule I, qui contient cependant encore la phénylalanine en BI, sous une forme cristalline.

3. Procédé pour la préparation d'une composition pharmaceutique, caractérisé en ce qu'on réunit un composé de formule I avec Y = acide aspartique sous une forme dissoute, avec un composé de formule I avec Y = aspargine sous une forme cristalline.